# EUROPEAN PATENT APPLICATION

(11) **EP 2 031 055 A1**
(43) Date of publication of application: **04.03.2009**
(21) Application number: 08170680.6
(22) Date of filing: 10.05.2002
(51) Int. Cl.: C12N 9/02

(54) **DNA molecule with insulator activity against chromosomal position effects in gene transfer processes in animal cells**

(30) Priority: 18.05.2001 ES 200101133
(62) Divisional of application: 02771658.8
(71) Applicant: Consejo Superior de Investigaciones Cientificas, 28006 Madrid (ES)
(72) Inventor: Montoliu José, Lluis Ctro. Nacional de Biotecnología, 28049 Madrid (ES); Giraldo Carbajo, Patricia Ctro. Nacional de Biotecnología, 28049 Madrid (ES); de Busturia Jimeno, Ana María Instituto de Biologica Molecular, 28049 Madrid (ES)
(74) Representative: Carpintero Lopez, Francisco

(57) **Abstract**

The invention relates to a DNA molecule or a fragment of said DNA molecule obtained from the LCR (Locus Control Region) sequence of the mouse tyrosinase gene. Said sequence has insulating activity against chromosomal position effects in gene transfer processes in animal cells. The DNA molecule can be used to introduce transgenes and to reduce the variability of the transgene expression in animal cells.

## Description

### AREA OF THE INVENTION

The invention refers to DNA molecules with insulator activity against chromosomal position effects in gene transfer processes in animal cells and the use of these to introduce transgenes and to reduce the variability of transgene expression in animal cells.

### BACKGROUND OF THE INVENTION

Gene transfer is a technique shared by many scientific disciplines, such as biology, biomedicine and biotechnology. The transfer of genes from their original position to a new location, which may not be suitable for the adequate expression of the experimental and/or therapeutic gene construct in question, is carried out to create a genetically modified animal for productive, biomedical or basic biological purposes, such as the development of vectors for gene therapy.

In general, almost all gene integration events associated with transfer processes (transgenesis, gene therapy etc) develop stochastically. The desired gene substitution or specific integration by homologous recombination processes is very rare and can only be effectively achieved in pluripotential mouse embryonic cells (ES), through specific selection processes. Unfortunately, this methodology can only be applied to mice, the only species for which pluripotential ES mouse embryonic cells capable of colonising all cell lines, including stem cells, have been isolated, permitting the alteration to be transferred to the offspring. Because of this, the generation of transgenic cells in other animal species apart from the mouse, and the application of gene transfer strategies in biotechnology and gene therapy in humans, is still largely dependent on the process being triggered by random integration.

The impredictability of the insertion of experimental and/or therapeutic sequences in the genome of the receptor organism has two consequences. In the first place, a possible but unavoidable risk of mutation by insertion in or interruption of the gene pattern. This first risk is not very large (partially due to the large proportion of uncoded sequences in mammal genomes) and, in mice, has been found to represent up to 7% of gene transfer events. Secondly, and proportionally much more important and responsible for the majority of failed and/or unexpected expression patterns in gene transfer processes, is the problem of position-effects, associated with the random site of insertion of the experimental and/or therapeutic construct in the genome of the receptor or host organism. If the experimental and/or therapeutic construct inserts close to regulatory elements of other genes it can be either negatively (in the case of *repressors* and/or silencers), or positively (in the case of *enhancers,* or transcriptional activators) affected, altering its expression pattern and obtaining results different to those initially planned, with possible ectopic expression (in unwanted expression sites) or at different times to those planned or to a lesser or greater degree than originally intended.

In eukaryote organisms, the genomes contain a large percentage of non coding intergenic sequences. In animals and, more specifically, in mammals such as mouse or man, these intergenic sequences can correspond to up to 95% of the genome. The remaining 5% contains exons (coding fractions) of the genes and adjacent regulatory regions. The genes are arranged consecutively, without any apparent organization. It is, especially, common to find genes with a totally different expression pattern located next to each other (for example a gene of hepatic expression next to one specific for cerebellum neurons). For some time it has been accepted that the best definition of gene locus is that of expression domain, which includes all coding and non-coding sequences, that enable the gene to be optimally expressed in appropriate time, place and amounts. The boundaries of the expression domains correspond to boundary elements or insulators the function of which is to control the interactions and influences of neighbouring expression domains to prevent the pattern of specific gene expression from being affected. Although one assumes that these insulator elements exist, for most (or perhaps all) of the expression domains very few sequences with these characteristics have been discovered and experimentally studied.

The existence of DNA insulator sequences, at the boundaries of expression domains, and their importance in the defining the structural and functional limits of the expression domains and their role as blockers of sequences of unwanted effects from outside the expression domain, was initially described in fruit flies (*Drosophila melanogaster*) via scs and scs' elements flanking the hsp70 gene pair (Kellum R & Schedl P. 1991. A position-effect assay for boundaries of higher order chromosomal domains. Cell 64:941-50; Kellum R & Schedl P. 1992. A group of scs elements function as domain boundaries in an enhancer-blocking assay. Mol. Cell Biol. 12 (5): 2424-31; Vazquez J & Schedl P. 1994. Sequences required for enhancer blocking activity of scs are located within two nuclease-hypersensitive regions. EMBO J. 13 (24): 5984-93; Geyer PK. 1997. The role of insulator elements in defining domains of gene expression. Curr opin Genet Dev. 7 (2): 242-8) who discovered one of the most surprising and essential characteristics of these elements: their potential to block the action of transcriptional enhancers when they are located between these and the gene promoter. After this, other *insulator* elements have been described in D. *Melanogaster* such as that of Su (Hw) (Geyer PK & Corces VG. 1992. DNA position-specific repression of transcription by a Drosophila zinc finger protein. Genes Dev. 6(10): 1865-73; Roseman RR et al., 1993. The su (Hw) protein insulates expression of the Drosophila Melanogaster white gene from chromosomal position-effects. EMBO. J. 12 (2): (435-42), other invertebrates, such as the element described in the arylsulphatase gene (Akasaka K et al., 1999. Upstream element of the sea urchin arylsulphatase gene serves as an insulator. Cell. Mol. Biol. (Noisy-le-grand ). 45 (5): 555-65) and the early histone cluster (Melfi R. et al. 2000. Functional characterization of the enhancer blocking element of the sea urchin early histone gene cluster reveals insulator properties and three essential cis-acting sequences. J. Mol. Biol. 304:753-763) of sea urchins and in vertebrates, such as the *insulator* of the chicken β-globin locus (Chung J. H. et al., 1993. A 5 ' element of the chicken β-globin domain serves as insulator in human erythroid cells and protects against position effect in Drosophila. Cell 74: 505-14; Chung J et al. 1997. Characterization of the chicken beta-globin insulator. Proc Natl Acad Sci USA. 94 (2): 575-80; Recillas-Targa F et al. 1999. Positional enhancer blocking activity of the chicken beta-globin insulator in transiently infected cells. Proc. Natl Acad Sci USA. 96 (25): 14354-9; Bell AC et al., 1999. The protein CTCF is required for the enhancer blocking activity of vertebrate insulators. Cell. 98 (3): 387-96). Nevertheless, none of these insulator sequences provide completely satisfactory results. Moreover, the use of chicken insulator DNA sequences (Chung et al., North American patent US 5.610.053) presents several problems including (i) the generation of transgenic animals difficult to reproduce with independent expression of position effects since, although there are examples that show that this has worked well (Taboit-Dameron et al., 1999. Association of the 5'HS4 sequence of the chicken-β globin locus control region with human EF1α promoter induces ubiquitous and high expression of human CD55 and CD59 cDNAs in transgenic rabbits. Transgenic research 8: 223-35) there are other examples that show that this has not worked as well as expected (Potts W et al. 2000. Chicken β-globin 5'HS4 insulators function to produce variability in transgenic founder mice. Biochemical and Biophysical Research Communications 273: 1015-8), and (ii) the need to multiply the presence of the insulator sequence on both sides of the gene construct that one wants to protect from the chromosomal position effects, that can cause cloning and stability problems and then induce inactivation of the host genome by repetition of tandem sequences at one site.

On the other hand, not all sites of the genome permit expression of the experimental and/or therapeutic construct to the same extent. There are euchromatic regions, in which the chromatin is exposed, little condensed and inaccessible to nuclear protein factors, which do permit expression. Others, called heterochromatic regions that have highly condensed chromatin and are inaccessible to nuclear protein factors do not permit gene expression. The presence of border elements or insulators in the experimental and/or therapeutic construct permit a possible heterochromatic region (not compatible with gene expression) to be reconverted into a euchromatic region (that permits gene expression).

In spite of the considerable advantages of being able to control chromosomal position effects in gene transfer processes in animal cells, there are only a limited number of DNA sequences with insulator activity, which also, in general, do not give completely satisfactory results. It is, therefore, still necessary to continue to look for new insulator DNA sequences in order to increase the number of ways to control position effects and to guarantee an optimum expression level of the experimental and/or therapeutic construct.

### SCOPE OF THE INVENTION

The invention relates to the problem associated with position effects that affect any gene transfer process and that cause, to a large extent, the lack of expression or the inadequate expression of experimental and/or therapeutic gene constructs, reducing in this way, to a variable degree, the success of these gene constructs.

The solution provided by the invention is based on the fact that the inventors have identified DNA molecules with insulator activity against chromosomal position effects in gene transfer processes in animal cells. The insulator activity of these DNA molecules has been demonstrated in experiments on the protection against position effects carried out in transgenic flies and mice.

A DNA molecule such as that provided by this invention, with insulator activity, permits position effects to be controlled limiting their effect, to guarantee an optimum expression level of the experimental and/or therapeutic construct in time, place and amount, regardless of the place where the experimental and/or therapeutic construct is inserted in the genome.

Therefore, these DNA molecules constitute an objective of this invention.

Another objective of this invention corresponds to a DNA construct that consists in, at least, one of these DNA molecules and a gene of interest.

Another additional objective of this invention corresponds to the use of these molecules or DNA constructs or these vectors, to produce recombinant animal cells, or to introduce transgenes into animal cells and/or to reduce the variability of transgene expression in animal cells. The animal cells and transgenic animals (excluding humans) are also objectives of this invention.

### BRIEF DESCRIPTION OF THE FIGURES

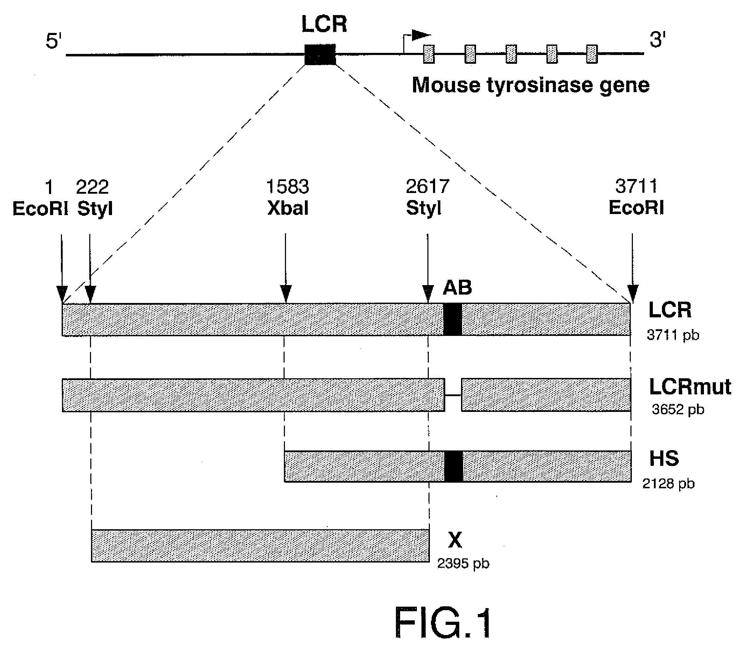

Figure 1 shows a diagrammatic representation of the DNA molecules called LCR, LCRmut, HS and X indicating their relative position in regulatory zone 5' of the gene expression of the mouse tyrosinase gene.

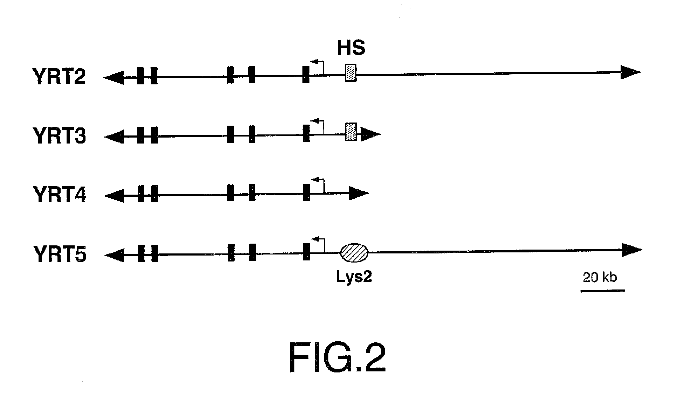

Figure 2 is a diagrammatic representation of the yeast artificial chromosomes (YACs) identified as YRT2, YRT3, YRT4 and YRT5 used to identify and verify locus controller activity of a molecule of mouse DNA obtained from the LCR (*locus control region*) sequence of the mouse tyrosinase gene (see Example 1).

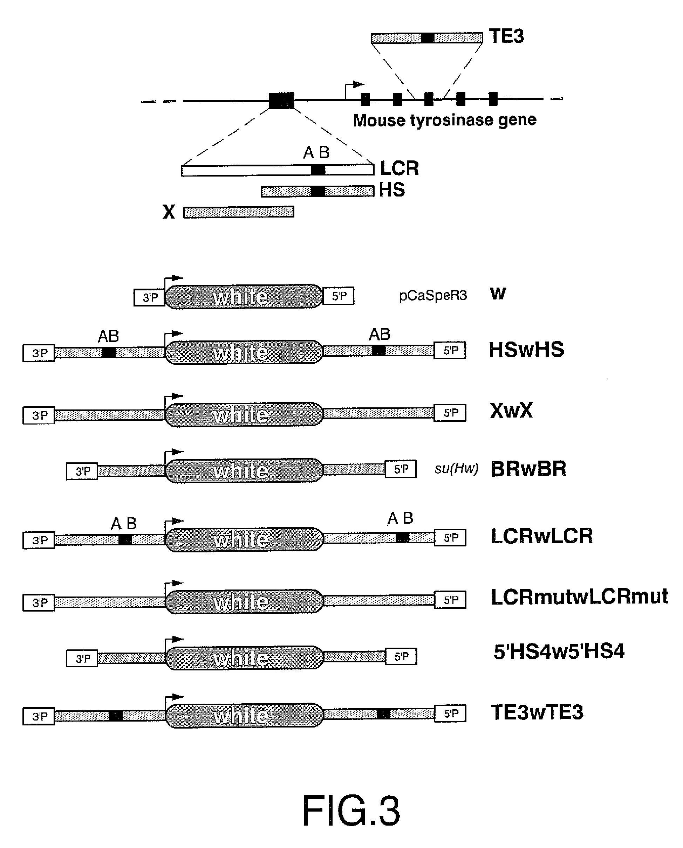

Figure 3 is a diagrammatic representation of a fragment of mouse chromosome 7 that contains the tyrosinase gene and its locus control region (LCR), and an amplified fragment of this LCR and of the region of the tyrosinase gene that contains the third exon and surrounding intronic sequences (TE3). Figure 3 also includes a diagram of the gene constructs used in a protection experiment against position effects in transgenic flies (see Example 2).

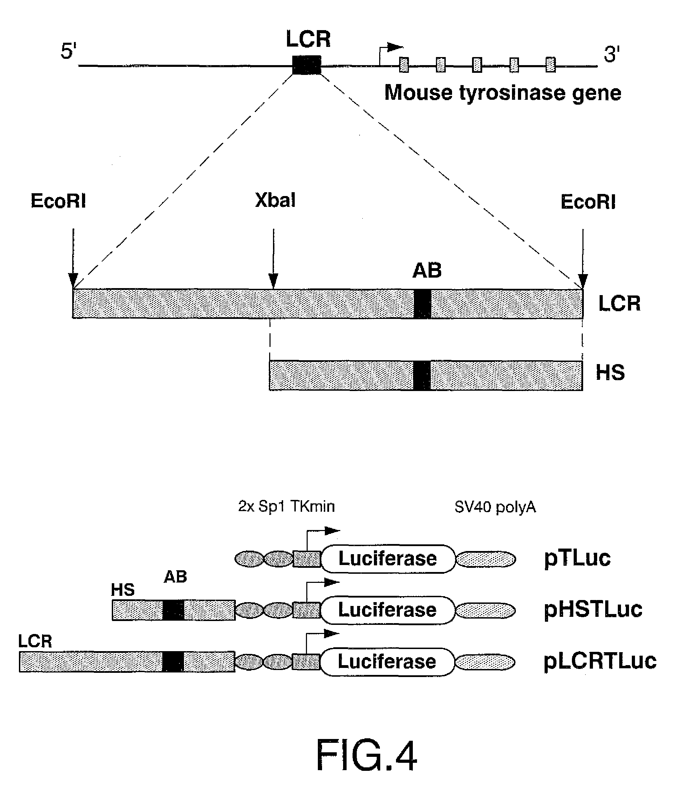

Figure 4 is a diagrammatic representation of a fragment of mouse chromosome 7 that contains the tyrosinase gene and a locus control region (LCR), and an amplified fragment of this LCR that shows the AB box and the HS fragment. The LCR and HS fragments shown in Figure 4 are identical to those that appear in Figure 1. Figure 4 also includes the gene constructs used in a protection experiment against position effects in transgenic mice (see Example 3).

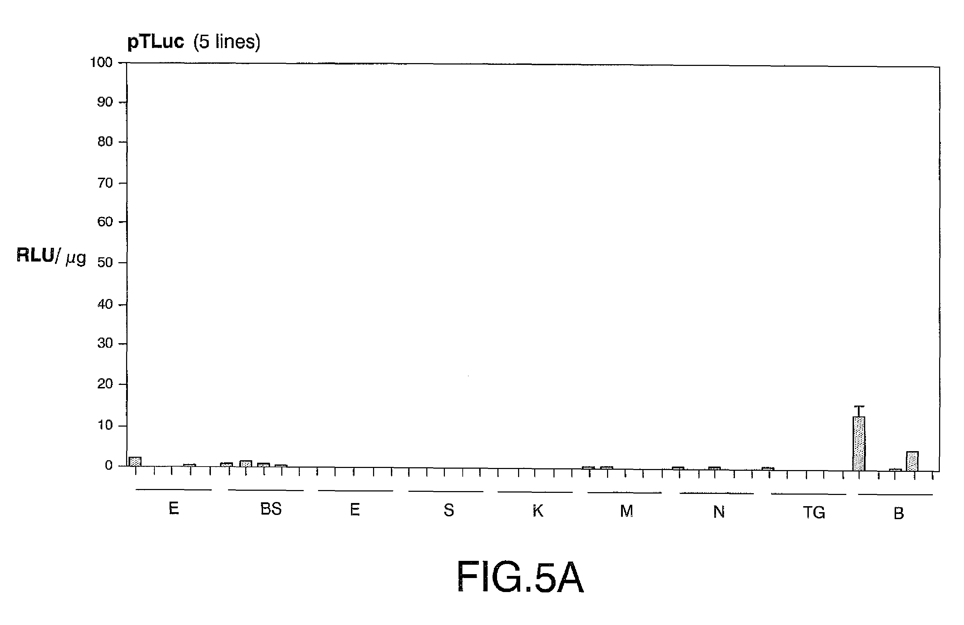

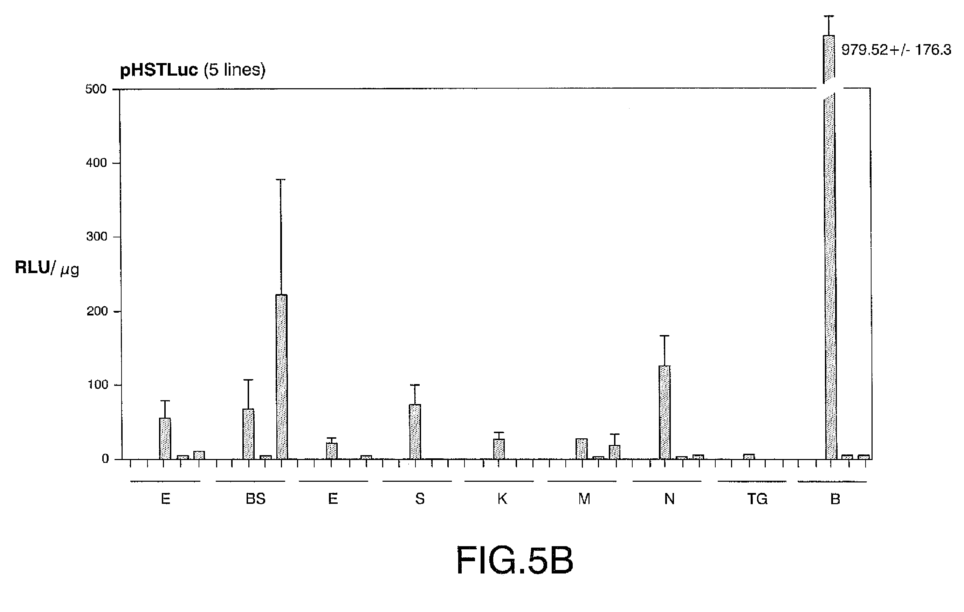

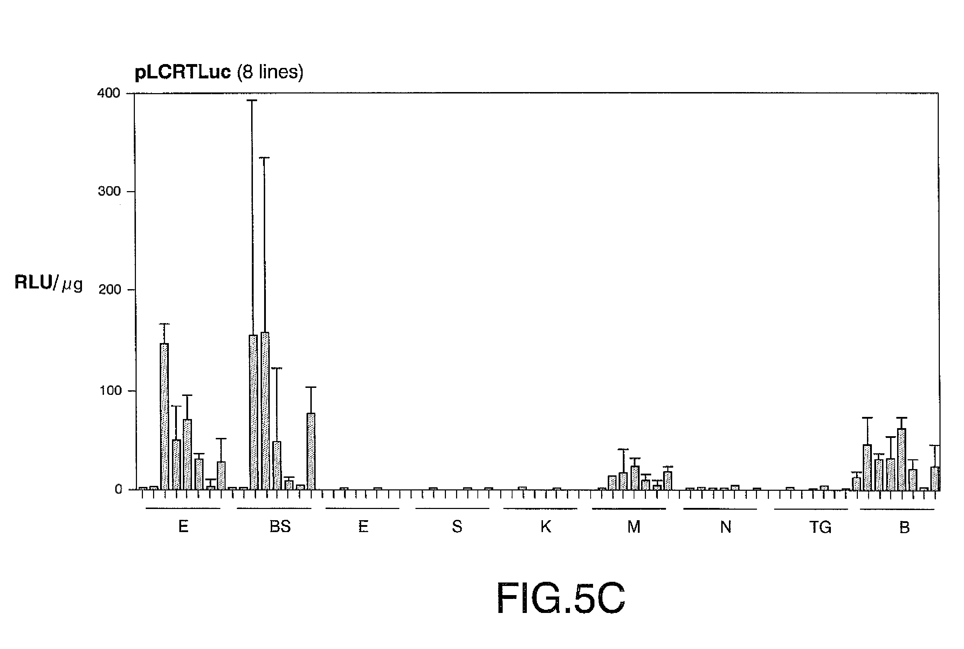

Figure 5 consists of three bar charts that show the activity of the luciferase indicator transgene (mean of six individuals +/- standard deviation) obtained in nine different tissues of different transgenic mouse lines obtained using the gene constructs called pTLuc (Figure 5A), pHSTLuc (Figure 5B) and pLCRTLuc (Figure 5C), shown in Figure 4. The abbreviations used are explained in the Material and Methods section of Example3.

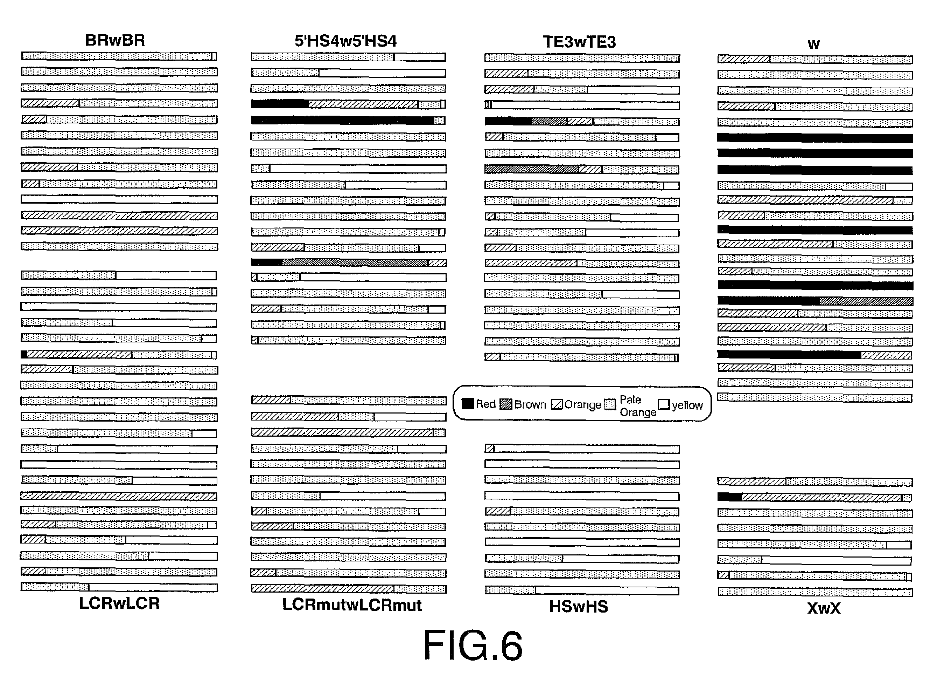

Figure 6 shows a set of independent lines of the transgenic fruit fly *Drosophila melanogaster* obtained using the DNA molecule with insulator activity provided by this invention, using the constructs described in Figure 3 [see Example 2]. Each horizontal bar represents an independent line. The data shown correspond to the analysis of eye colour in heterozygote individuals of transgenic flies, i.e. in carriers of one copy of the construct. Each bar shows the percentage of flies with each of the five categories of eye colour [*red (=wild-type), brown, orange, pale orange* and *yellow*].

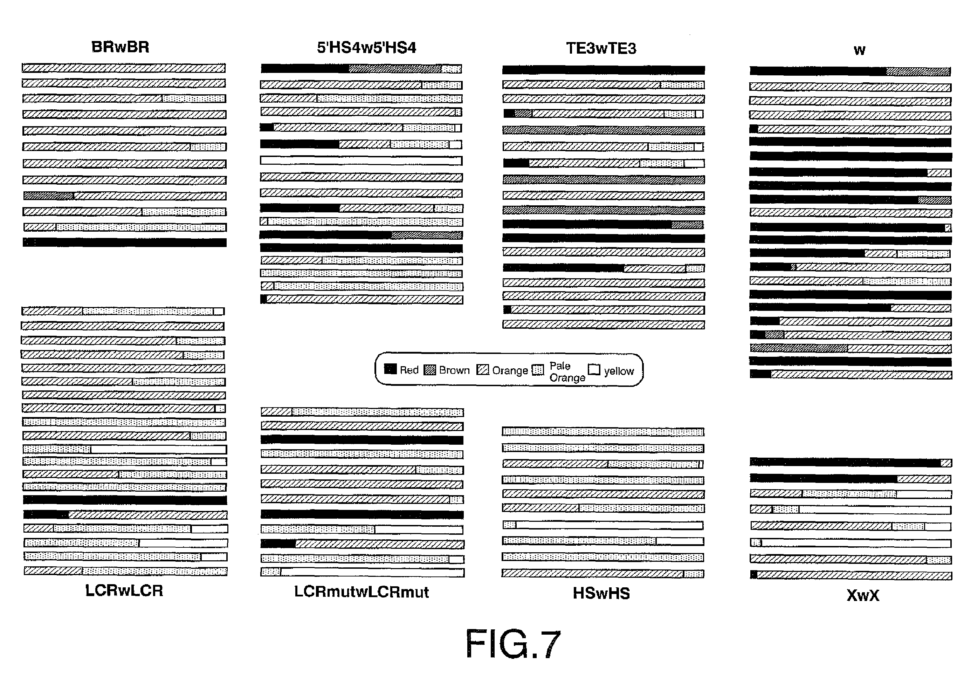

Figure 7 shows the same set of independent lines of transgenic *Drosophila melanogaster* flies shown in Figure 6 and obtained using a DNA molecule with insulator activity provided by this invention using the constructs described in Figure 3 [see Example 2]. In this case, the data shown correspond to the analysis of eye colour of transgenic flies in homozygote individuals, i.e. carriers of two copies of the construct, one in each homologous chromosome. Each horizontal bar represents an independent line. Each bar graphically represents the percentage of flies with each of the five categories of eye colour considered [*red (= wild-type), brown, orange, pale orange* and *yellow*].

### DETAILLED DESCRIPTION OF THE INVENTION

The invention provides a DNA molecule, from hereon called the DNA molecule of the invention that consists in a nucleotide sequence selected from:
a) the nucleotide sequence identified as SEC. ID. Nº:1, or a fragment of this, and
b) an analogous nucleotide sequence to that defined in a).

In the sense used in this description, the term "analogous" aims to include any DNA sequence that can be isolated or constructed using the nucleotide sequence shown in SEC.ID. No:1, for example, by introducing conserved or non conserved nucleotide substitutions, including the insertion of one or more nucleotides, the addition of one or more nucleotides at either end of the molecule or the deletion of one or more nucleotides at either end or inside the sequence.

In general, an analogous DNA molecule is substantially homologous to the nucleotide sequence identified as SEC. ID. Nº:1. In the sense used in this description, the expression "substantially homologous" means that the nucleotide sequences in question have a degree of identity on the nucleotide level of , at least, 70%, preferably of at least, 85% or even preferably of , at least, 95%.

The DNA molecule of the invention can proceed from an organism in which it is found naturally or from a host organism transformed with this DNA molecule. The DNA molecule of the invention can be isolated, by conventional techniques, from the DNA of any organism that contains it using probes or oligonucleotides prepared from information about the nucleotide sequence of the DNA molecule provided in this description.

The DNA molecule of the invention presents insulator activity from chromosomal position effects in gene transfer processes in animal cells. The insulator activity of the DNA molecule of the invention can be studied by protection experiments from position effects carried out in transgenic flies and mice that are described in Examples 2 and 3. The DNA molecule of the invention includes fragments of it with this insulator activity.

In one specific application, the DNA molecule of the invention is a molecule of mouse DNA obtained from the LCR sequence (*locus control region*) of the mouse tyrosinase gene, specifically, it corresponds to the *EcoRI-EcoRI* restriction fragment of the regulator region 5' of the tyrosinase gene (*mouse genome informatics* (MGI) code) 98880 The Jackson laboratory USA), situated at approximately twelve thousand kilobases (-12kb) from the start of transcription of the tyrosinase gene, located in the distal end of murine chromosome 7, in position 44 cM of the genetic map of this chromosome. This DNA molecule is 3,711 base pairs (bp) long and its nucleotide sequence corresponds to SEC. ID. Nº:1. Of this DNA molecule, the 1,133 bp of its 3'terminal region are known (*GenBank* X76647) (Ganss R et al. 1994 EMBO J. 13: 3083-93).

The insulator activity of this DNA molecule has been determined experimentally both in the complete sequence of 3,711 bp called LCR (SEC.ID Nº:1) and in different smaller fragments included and/or derived from this called (see Figure 1):
LCRmut: identical to LCR except for a 59 bp deletion that eliminates the AB box between positions 2622 and 2683, including SEC. ID. Nº:1 (3,652 bp);
HS: corresponding to the nucleotide sequence between nucleotide 1583 and 3711 of the SEC.ID. Nº:1 (2128 bp); and
X: corresponding to the nucleotide sequence between nucleotide 222 and 2,617 of the SEC.ID Nº:1 (2,395 bp).

The DNA molecules LCR (SEC. ID. Nº1), LCRmut, HS and X have shown insulator activity in experiments of protection from position effects carried out in transgenic fruit flies (*D. Melanogaster*) [see Example 2]. Moreover, the DNA molecules LCR (SEC: ID. Nº.1) and HS have equally been shown to have insulator activity in experiments on protection from position effects carried out in transgenic mice [see Example 3].

The DNA molecule of 3,711 bp called LCR (SEC: ID. Nº:1) was initially identified as an *EcoRI-EcoRI* fragment that contains hypersensitivity to the DNase enzyme specific to mouse melanoma cells, that expresses the tyrosinase gene (Ganss et al. 1994. EMBO J. 13:3083-93). The hypersensitivities to cleavage by the DNasel enzyme are usually associated with the presence of specific DNA sequences for binding to nuclear protein regulators of gene expression such as the transcription factors. The search for new gene regulator regions, such as the DNA molecule called LCR, situated twelve thousand base pairs from the start of transcription of the tyrosinase gene, was started to try to understand the different behaviour of transgenes with the mouse tyrosinase gene based on minigenes (Beerman et al., 1990. EMBO J. 9:2819-2826) or yeast artificial chromosomes (Schedl et al., 1993. Nature 362: 258-261). The former limit the presence of 5'regulators of the tyrosinase gene to around 5 kb and only contain the first intron. The second contain up to 150 kb of regions that are potential regulators of 5' and all the introns that space the five exons of the mouse tyrosinase gene.

The use of minigenes with the tyrosinase gene produced a variable pigmentation level in transgenic mice lower than that obtained in wild type mice (Beerman et al. 1990. EMBO J. 9:2819-2826). In contrast, the use of yeast artificial chromosomes guaranteed a pigment indistinguishable from that of wild type mice in all independent lines of transgenic mice studied (Schedl et al., 1993. Nature 362:258-261). The clear difference in the expression potential of both types of gene construct suggests the existence of new DNA sequences of regulators of gene expression, present in the artificial chromosomes of yeast and absent from the minigenes, responsible for the correct functioning of the transgene in transgenic mice.

The first indication of the existence of a possible regulator region situated beyond the 5 kb analysed in the tyrosinase minigenes arose from the characterization of the *chinchilla-mottled* allele of the tyrosinase gene, which has a variegated pigmentation associated phenotype. The investigators detected a loss of hypersensitivity (HS) to the DNasel around 15 kb from the start of transcription of the gene and postulated that this absence was the molecular basis of the mutant allele mentioned (Porter et al., 1991 Dev. Genetics 12:393-402). After, it was shown that this HS region is included within the *EcoRI-EcoRI* fragment called LCR throughout this document and represented by SEC. ID. Nº:1. After, it was shown that deletion of the HS region in yeast artificial chromosomes was associated with loss of the wild-type pigmentation and the appearance of a much weaker and variegated pigmentation (Montolieu et al., 1996 EMBO J. 15: 6026-6034) [see Figure 2 and Example 1]. From these results, a DNA molecule 3711 base pairs long was identified (SEC ID. Nº.1) as a locus control region (LCR) of the mouse tyrosinase gene, analogously to the definitions made for other LCRs identified functionally in transgenic animals (Li et al., 1999 Trends Genet. 15: 403-408).

In this 3,711 bp DNA molecule, an enhancer function of gene expression (the activity of which depends on the presence of the AB box, see Figure 1) (Ganss et al., 1994 EMBO J. 13: 3083-93) and an enhancer function of position effects, described in this document, coexist, independent of the presence of the AB box (see Figures 1 and 3 and Example 2; compare the insulator activity obtained with the LCR or LCRmut molecule).

Although we do not want this to be associated with any specific hypothesis, analysis of the insulator activity of the sequences studied (LCR, LCRmut, HS and X) suggest the presence of a common fragment to all of them of 1,034 bp, delimited by the enzymes *XbaI* (in position 1,583) and *styII* (in position 2,617), that could be responsible for this insulator activity.

The invention also provides a DNA construct, from hereon called the construct of the invention, that consists of the DNA molecule of the invention. The construct of the invention can be used to isolate and protect the genes and to produce proteins or products of interest encoded by the gene or gene(s) used in these constructs. The presence of, at least, one DNA molecule of the invention in the DNA construct of the invention permits position effects to be controlled, limiting their effects, and guaranteeing an optimum expression level of this gene construct in time, place and quantity, regardless of the place where the gene construct is inserted in the genome of the animal host or receptor organism used.

In general, the DNA construct of the invention consists in, at least, one DNA sequence of the invention and a transcription unit that contains, at least, one gene of interest, such as a gene that encodes a protein or its precursor, or a product of interest, and a promoter that directs transcription of the gene of interest, to which it is operatively bound, and other necessary or appropriate sequences for transcription of the gene of interest and its appropriate regulation in time and place, for example start and stop signals, cleavage sites, polyadenylation signal, replication origin, transcriptional enhancers, transcriptional silencers etc.

The DNA construct of the invention can contain one or more DNA molecules of the invention. In one specific application, the DNA construct of the invention contains, at least, 2 DNA molecules of the invention in tandem form with direct and/or inverted orientations since the function of these is independent of their orientation relative to the transcription unit. The DNA molecules of the invention are situated such that the gene of interest is insulated from the DNA that surrounds the integration site. Preferably, the DNA molecules of the invention are situated on both sides of the gene of interest and protect it from the effects of the DNA sequences that operate in cis of the chromatin.

In one specific application, appropriate to isolate expression of the gene of interest, a first DNA molecule of the invention is situated in position 5' (*upstream*) of the transcription unit and a second DNA molecule of the invention, that can be the same as or different from the first one and can have the same or a different orientation, is situated in position 3' (*downstream*) of the gene of interest. In the case that the DNA construct of the invention has more than one gene of interest, the transcription units of these genes can be separated by DNA molecules of the invention.

Gene of interest refers to any transcription unit that codes for a protein with a biological, therapeutic or biotechnological activity that is interesting and relevant for society. Examples of these, but in no way limiting to these, are blood coagulation factors VIII and IX, the absence of which causes Type A or Type B congenital hemophilia, respectively; the gene that codes for the CFTR protein, the absence of which causes cystic fibrosis, the most common autosomally inherited congenital disease; the gene that codes for growth hormone, required in patients with different types of dwarfism etc. Genes of interest include those to be expressed under the control of DNA sequences of specific expression in the mammary gland, with the aim of being able to produce and purify recombinant protein in the milk of transgenic animals. Sequences of specific DNA expression in the mammary gland exist and are known (whey acid protein [WAP], the α-lactoglobin gene, the α-lactalbumin gene, casein genes) although all of these, to a greater or lesser extent, are exposed to position effects in gene transfer experiments and could benefit from the addition of insulator sequences such as that described in this report. Also, the genes of interest include any transcription unit for which one wants to study the function over development and, in general, in any experimental situation that concerns any gene transfer process for which the objective is the biological study of the gene in question.

The DNA constructs of the invention can be used, in general, in any gene transfer process, for example, to generate transgenic animals for biological, biomedical and/or biotechnological purposes, to generate expression vectors for gene therapy based on viral and non viral elements that require insertion of the therapeutic DNA in the host organism genome, and, in general, in any application that requires gene transfer and for which the level and expression pattern must be guaranteed optimally, regardless of the site of insertion in the host organism genome. The DNA molecule of the invention must also be used to isolate specific genes introduced and expressed later in transgenic animals such as flies, mice etc. The DNA constructs of the invention can be introduced in fetal cells or in embryos to produce transgenic animals that contain the DNA molecule of the invention and the desired gene of interest. By insulating the gene of interest introduced in the transgenic animal, expression of this gene is protected against its negative or inappropriate positive expression.

DNA constructs of the invention can be inserted in appropriate vectors, such as plasmids, yeast artificial chromosomes (YACs), bacterial artificial chromosomes (BACs), artificial chromosomes based on the bacteriophage P1 (PACs), cosmids or viruses, that can also contain a bacterial origin or of replicator yeast, and a marker to be used to select transfected cells other than the gene or genes of interest. Therefore, the invention also refers to a vector that consists of a DNA construct of the invention. The choice of vector will depend on the host cell in which this is to be later introduced. As an example, the vector in which this DNA sequence is introduced can be a plasmid which, when it is introduced in a host cell integrates in the genome of this cell and replicates together with the chromosome (or chromosomes) in which either a virus, or, for example, a retrovirus useful in gene therapy is introduced.

The vector of the invention can be obtained by conventional methods known by experts in the area (Kovesdi et al. 1997. Curr. Opin. Biotech. 8: 583-589; Giraldo and Montoliu, 2001. Transgenic Res. 10:83-103; Coffin et al., 1998. Retroviruses, CSHLP, Robbins et al., 1998. Trends Biotech. 16 : 35-40 ; Anderson. 1998. Nature 392 : 25-30 ; Schindelhauer. 1999. Bioessays 21 : 76-83).

The invention also provides an animal cell that contains a DNA molecule of the invention, or a DNA construct of the invention or the abovementioned vector. The host cells that can be transformed with the DNA construct of the invention or with this vector can be, for example, eukaryotic cells, such as cells of animal tissues and animal cell lines (Douglas et al., 1996. Nature Biotech. 14: 1574-1578); Guidotti et al., 1998. Hum. Mol Gen 7: 831-838; Huxley. 1997. Trends Genet. 13: 345-347; Rols et al., 1998. Nature Biotech 16: 168-171). The transformation of animal cells can be done by conventional methods (Ausubel et al., eds. 1990. Current Protocols in Molecular Biology, Green Publishing Associates and Wilry Interscience).

The invention also provides a transgenic animal that contains at least one transgenic animal cell that contains, at least, one DNA molecule of the invention or a DNA construct of the invention or the above mentioned vector. The term "transgenic animal" does not include humans, although human cells, especially non germinal cells, can be transformed with the DNA molecule, the construct or the vector provided by this invention in any of the variants of so-called somatic gene therapy.

The invention also provides a kit that consists in, at least, one DNA molecule of the invention or a DNA construct of the invention or a vector provided by this invention. In one specific application, this DNA molecule of the invention can be contained in a vector or plasmid that contains, or can be easily adapted by the user, the appropriate DNA elements for adequate expression of the gene or genes of interest. As an example, the vector or plasmid can contain, at least, one DNA molecule of the invention and, at least, one insertion site of the gene of interest or of its transcription unit, defined by a region of restriction enzyme recognition. The plasmid or vector contained in these kits can be immersed in a stabilisation and conservation medium of the kind commonly used for this type of product, or can be lyophilised and reconstituted when it is to be used by adding a suitable medium.

Moreover, the invention provides a method to produce recombinant animal cells, or to introduce transgenes in animal cells, or to reduce the variability of transgene expression in animal cells, that corresponds to using a DNA construct of the invention, more specifically, that consists in transforming animal cells with this DNA construct of the invention under conditions in which these cells can be transformed.

The following examples illustrate the invention.

### EXAMPLE 1

### To obtain and characterise DNA molecules of murine origin with LCR activity

A DNA molecule with insulator activity was obtained from the LCR sequence (*Locus Control Region*) of the tyrosinase mouse gene. Tyrosinase is known to be a key enzyme in melanin biosynthesis. The absence or defective synthesis of this enzyme is the commonest cause of albinism, a condition described in mammals and in most vertebrates. The gene is regulated during development and has a very specific expression pattern only being expressed in two types of cells not related ontogenetically: melanocytes present in the skin, capillary follicles, iris and choroids, and in the pigmented epithelium of the retina. The first are derived from the migration followed by the differentiation of neural crest cells whereas the pigmented epithelium of the retina is derived from the eye cup.

The mouse tyrosinase gene corresponds to the c or "albino" locus and is situated in murine chromosome 7. The tyrosinase gene in the mouse is composed of five exons that occupy around one hundred kilobases (kb) of the genome. Several research groups have simultaneously described that the molecular basis of albinism in mice was a point mutation that modified an amino acid of the protein, transforming it into an inactive product. The identity of tyrosinase, with the albino c locus, was finally confirmed when transgenic pigmented mice were obtained that were carriers of functional copies of the tyrosinase gene, capable of correcting the albino condition in microinjected embryos. Nevertheless, the level of pigmentation obtained with these minigenes was highly variable and in no case was comparable with that of the wild-type strain, suggesting that position effects were probably the most likely cause of deficient transgene expression (Beerman et al., 1990. EMBO J. 9:2819-2826). The use of tyrosinase transgenes based on YACs permitted normal expression of the gene to be recovered obtaining a complete correction of the albino phenotype (Schedl et al., 1993. A yeast artificial chromosome covering the tyrosinase gene confers copy-number dependent expression in transgenic mice. Nature 362: 258-261; Jeffrey G et al., 1994. Correction of abnormal retinal pathways found in albinism by introduction of a functional tyrosinase gene in transgenic mice and rabbits. Brain Res Dev Brain Res. 99: 95-102). As a result, the tyrosinase gene system and the correction of albinism is an excellent experimental model to study the mechanisms involved in gene transfer processes since an autonomous expression domain is available (250 kb in a YAC) capable of organising the chromatin suitably regardless of the site of insertion in the receptor genome.

In the case of the tyrosinase gene the comparison between transgenes based on minigenes or on YACs indicated that the latter contained all the regulatory elements of gene expression, elements that were absent from the original constructs. The transgenes based on YACs were able to reconstruct the original expression pattern of the endogenous gene wherever this was integrated. All this shows that some of the elements described previously that were not included in the minigenes, must also be present in the YAC.

The YAC used in the trials carried out by the inventors had around 155 kb of promoter and adjacent 5' regions where the element or elements could exist. One indication to find this element arose from a work that described the molecular basis of a c locus allele (*chinchilla-mottled)* characterised by the striped pattern of carrier mice. A possible insertion or inversion of sequences was described that translated a hypersensitive site to the Dnasel (HS) of its usual site (at around 12 kilobases in the 5' direction of the promoter) at tens of kilobases from the promoter (Porter S. et al., 1991.Mosaicism of tyrosinase-locus transcription & chromatin structure in dark vs light melanocyte clones of homozygous chinchilla-mottled mice. Dev. Genet., 12, 393-402.). This sequence contained a specific HS of melanoma cells (cells that express tyrosinase). Moreover, analysis by cellular transfection detected that this HS behaved as a potent transcriptional enhancer and, by deletions, the activity was limited to a sequence of 200 bp that seemed to join nuclear proteins (*in vitro*) (Ganss et al., 1994. A cell specific enhancer far upstream of the mouse tyrosinase gene confers high level and copy-number related expression in transgenic mice. EMBO J. 13: 3083-93). In this same work it was demonstrated that addition of this HS sequence to one of the mouse tyrosinase minigenes used previously improved its behaviour in transgenic animals conferring it an expression level relative to the number of copies integrated in the transgenic mouse genome (Montoliu 1994. Current Biol. 4:1025).

Nevertheless, the final result that permits this HS sequence to be associated with a locus control region (LCR) was obtained by combining the new technology of transgenic mice with YACs and the infinite possibilities for modification offered by the yeast system. Hence, three new YACs were constructed from the original 250 kb, called YRT2. In the first place (and always using homologous recombination techniques in yeast) all the sequences situated beyond the recently identified HS, situated around 12 kb above the promoter, were deleted. In this way, a smaller YAC was generated (around 100 kb) than the one called YRT3. In the second place, a new deletion was prepared that included the HS sequence, resulting in the disappearance of this from the YAC, generating a different YAC to the one called YRT4. Finally, the HS sequence was replaced by a yeast gene marker obtaining the YAC called YRT5, identical to the original YRT2 but without the HS sequence. Figure 2 shows diagrammatically the transgenes (YRT2, YRT3, YRT4 and YRT5).

The transgenes YRT3, YRT4 and YRT5 were microinjected to albino mice embryos and the transgenic mice obtained were studied. The result was unmistakeable. Mice with transgene YRT3, that maintained the HS sequence but lost all the sequences beyond the 5'end of the HS sequence, were pigmented as wild type mice or like mice with the original YRT2 transgene. However, both a deletion and substitution of the HS sequence corresponded to a very important loss in the level of pigmentation achieved. Transgenic mice with YRT4 and YRT5 presented a variable and much weaker expression level (variegated). Often, the pigment was only visible in the eyes although afterwards variegated expression of the YRT4 tyrosinase transgenes was demonstrated in all cell types capable of expressing this gene (Gimenez et al., 2001. Variegated expression and delayed retinal pigmentation during development in transgenic mice with a deletion in the locus control region of the tyrosinase gene. Genesis 30 (1): 21-25). These repeatedly verified results reflect the importance of this HS sequence in its behaviour as an LCR since no other surrounding sequence was able to compensate for this in its absence nor did it require others to efficiently command transcription of the gene (Montoliu L. et al., 1996. A locus control region at -12 kb of the tyrosinase gene. EMBO J 15: 6026-6034).

### EXAMPLE 2

### Generation of transgenic flies with the potential to guarantee protection of an experimental gene construct against chromosomal position effects in the fly genome

### Material and methods

### Constructs used in transgenic flies (see Figure 3).

**w (pCaSpeR3):** described in Pirrota, V. 1993, in R. L. Rodrigues and D. T. Denhardt (eds) Vectors: A survey of Molecular Cloning. Vectors and their uses, p. 437-456. This is the control plasmid of reference, from which all other gene constructs are derived.

**BRwBR:** Plasmid B.R.>w>B.R. described in Roseman et al., 1993. The EMBO Journal 12 2:435-442,. This includes a known sequence of *Drosophila Melanogaster* with insulator activity, initially described in the *gypsy* element. It is used as a positive control of insulator sequence.

**pCaSpeRW15*:** plasmid pCaSpeRW15 (Roseman et al., 1993. The EMBO Journal 12 2: 435-442) modified in its polylinker. This is not used to generate transgenic flies but forms the basis of clonings of experimental constructs. To obtain it, the 1.8 kb band obtained by digestion of the pCaSpeR3 is cloned in the vector pCaSpeRW15 used as a control in the presence of an *EcoRI* site present in 3' of the *white* minigene, required to obtain the experimental constructs.

**5'HS4w5'HS4:** The 5'HS4 fragment of the chicken β-globin gene (Chung et al., 1993. Cell 74: 505-514) in two copies (1.2 kb x 2 = 2.4 kb) is obtained by digestion with *XbaI* of the plasmid pBC1 (Invitrogen) and is cloned in a Bluescript plasmid (BS KSII+, Stratagene) to be able to transfer it now as a *BamHI-NotI* band that is inserted in 5' of the *white* minigenes of plasmid pCaSpeRW15* also digested with *BamHI-Not*I. For cloning in 3' of the *white* minigene blunt ends of the band and the vector pCaSpeRW15* are made, digested with *EcoRI*. This construct is a positive control, with a known vertebrate sequence (of chicken) with insulator activity.

**XwX:** The X fragment is obtained from the pTyr14:E6 plasmid (Ganss et al., 1994. the Embo Journal 13, 13:3083-3093) digested with *StyI* / (see Figure 1) and cloned in the *XbaI* site of the pCaSpeRW15* after generating blunt ends in both DNA fragments. The plasmid obtained contains the X fragment in 5' of the *white* minigene. To include the X fragment in the 3' region of the *white* minigene this is digested with *EcoRI*, blunt ends are made and the same X band is cloned at the site used in 5' but with blunt ends.

**HSwHS:** The HS region is obtained from the pTyr14:E6 plasmid (Ganss et al., 1994. The EMBO journal 13, 13: 3083-3093) digested with *XbaI-EcoRI* (see Figure 1), blunt ends are made and this is cloned on the bluntened *XbaI* site of the pCaSpeRW15*. In this step an *EcoRI* site is generated at the end of the HS region that is eliminated by partial digestion of the plasmid and selection of those molecules that have eliminated it and that present a unique *EcoRI* site at the end of the *white* minigene in which this is cloned once again the HS zone by digestion of the plasmid with *EcoRI* and generation of blunt ends.

**TEwTE3:** The TE3 fragment of 1.7 kb is obtained from the plasmid pgTYR-H1, 8A (Ruppert et al., 1988. EMBO J 7: 2715-2722) by digestion with *HindIII ,* blunt ends are made and the bluntened *XbaI* of pCaSpeRW15* is cloned in the site. The same band is then cloned after in the bluntened *EcoRI* site of the plasmid generated. This construct contains the third exon of the tyrosinase gene together with surrounding intronic sequences (see Figure 3) as a negative control of the experiment.

**LCRwLCR:**The LCR fragment is obtained from the plasmid pTYR14:E6 (Ganss et al. 1994. EMBO J 13.3083-3093) digested with *EcoRI*, blunt ends are made and this is cloned in plasmid pCaSpeRW15* digested with *ECO-RI* for directed cloning of the LCR band.

**LCRmutwLCRmut:** The LCRmut fragment obtained from the LCR fragment by partial digestions that manage to eliminate the DNA sequence between the sites *StyI-DdEI* that contain the AB box (see Figure 1). The resulting fragment is cloned in a Bluescript vector (BS KSII+, Stratagene) for its manipulation. The process followed to obtain the construct LCRmutwLCRmut is analogous to that described for LCRwLCR.

**To process obtain transgenic flies:**

The process followed to obtain transgenic fruit flies (*Drosophila melanogaster*) was carried out basically as described by Rubin and Spradling 1982 (Science, 218. 348-353). A solution was prepared in microinjection buffer (10 mM Tris-HCl pH = 7.5, EDTA 0.1 mM pH = 8) of the construct DNA at a concentration of 0.4 mg/ml and plasmid DNA pn25.7 at a concentration of 0.1 mg/ml that contains the gene that codes for the transposase, an enzyme required for the process of DNA insertion into the fly genome. This resulting solution, conserved at 4ºC, is the one that is injected into the fly embryos.

The *Drosophila melanogaster* strains used as hosts were, in this case, w1118 or Df ac1 w1118, carriers of the same inactivator mutation of the *white* gene (Bloomington Stock Centre, Indiana University, USA; http://flystocks.bio.indiana.edu/). The flies are placed in laying cells the night before, with yeast in the agar plate. It is recommendable to use fertilised young flies. In the morning, the plates are changed and the flies are left at 25ºC in the dark for half an hour. After this time the eggs are collected using water and a paintbrush. To do this, water is poured onto the plates until the embryos become unattached, this is then poured onto a filter connected to a vacuum to remove the liquid. After drying, the embryos are rinsed on the filter for two minutes with bleach to remove the chorion and washed three times with water.

After this the process is carried out in a heat-controlled room kept at 18ºC so that the embryos develop more slowly than at room temperature. The embryos are arranged so that their micropyles are all facing the same direction. After arranging them they are placed on the slide of an optical microscope previously impregnated with an adhesive glue made from heptane and double-sided adhesive tape (Sellotape). After this, the embryos are dried for 20 minutes in silica gel at a temperature of 20-22ºC.

A needle, made by lengthening a capillary tube, is loaded with DNA. The needle is placed under the microscope and the tip is broken off on the side of the coverslip fixed to a slide, with immersion oil. After drying the embryos they are covered with immersion oil, care is taken not to use too much immersion oil to prevent the future larvae from drowning. The slide with the eggs is placed under the microscope and the side opposite to the micropyle is injected. To inject DNA the needle must be thin enough not to rupture the embryo.

After injecting the DNA the microscope slide is removed and left on an agar plate at room temperature. The slides that contain the injected embryos are left at room temperature and the larvae collected after 24h. These are then transferred to tubes containing feed with a maximum of 100-110 larvae per tube and stored in a chamber at 25ºC until the flies begin to emerge. When this occurs, the Go generation is collected, separating the males from the virgin females that are crossed individually with flies of the host strain to detect transforming (transgenic) individuals.

Transgenic P[w] individuals are obtained, a homozygote stock is established and chromosomal localisation of the insertion is carried out. This is done by crossing the males P[w] with females w1118; If/CyO; TM2/MKRS (see Lindsley and Zimm. 1992. The Genome of Drosophila melanogaster. Academic Press Inc. San Diego, CA, for the significance of the markers).

For the analysis of eye colour in transgenic mice in heterozygosis, males of homozygote stock are crossed with virgin female of the w1118 stock. Between 30 and 50 female virgins of the F₁ are generated and separated into phials with food for observation of eye colour after 72 hours. The eye colour is recorded and each individual is assigned to one of the following categories of eye colour: (from greatest to least pigmentation intensity): *red (=wild type), brown, orange, pale orange* and *yellow.*

For the analysis of eye colour in transgenic mice in homozygosis, between 30 and 50 female virgins of the homozygote stock are used that are selected and separated into phials with food for observation of eye colour after 72 hours. The eye colour is recorded and each individual is assigned to one of the following categories of eye colour: (from greatest to least pigmentation intensity): *red (=wild type), brown, orange, pale orange* and *yellow.*

A protection experiment has been carried out on the position effects in transgenic flies, using the *white* minigene (that confers colour to fly eyes) on *white* flies (mutants, with white eyes). The *white* minigene has a limited expression potential (it gives a yellowish-orange colouration) but is used in gene transfer experiments since it is very effective at "reading" the position effects of the site of insertion in the fly genome (see for example: Chung et al., 1993, cited *supra;* Roseman et al., cited *supra;* Vazquez & Schedl 1994, cited *supra;* Namciu et al., 1998. Mol. Cell Biol. Human matrix attachment regions insulate transgene expression from chromosomal position effects in Drosophila melanogaster. 18.2382-2391). The position effects detected are mainly of the enhancer type (eye colour is enhanced above the capacities of the minigene since this remains in the proximity of a positive regulator element of the mouse genome). An insulator sequence that protects against these position effects will give the transgene back its real expression potential, insulating it from the influx of neighbouring sequences. Therefore, its eyes appear lighter and closer to *yellow* or *pale-orange.*

To assess the position effects, 8 gene constructs were used (see Tables 1 and 2 and Figures 3, 6 and 7). In heterozygote individuals (see Table 1 and Figure 6), the first reference (w), only contains the *white* minigene. The 24 independent transgenic fly lines obtained with w and analysed vary in colour from *yellow* to *red.* As a negative experimental control, exon 3 and its adjacent intronic sequences of the mouse tyrosinase gene (TEwTE3) were used. In this case, the 20 independent lines of transgenic flies obtained also varied in colour between *yellow* and *red,* no significant protection was obtained due to mouse sequences unrelated with the experimental LCR region, but belonging to the same mouse tyrosinase gene.

As positive controls of the experiment, two well known insulator sequences were used: the 5'HS4 [with the *insulator* of the chicken β-globin locus (Chung et al. 1993, cited *supra*)] and the BR [with the *insulator* of the *Suppressor of Hairy Wing* of the gypsy retrotransposable element in *D. Melanogaster* (Roseman et al., 1993, cited *supra*)]. The first of these, 5'HS4w5'HS4, carries a duplication of the 1.2 kb element described on each side of the *white* minigene. A displacement is observed, in number, of the 19 transgenic fly lines studied, to lighter colourations (due to protection against position effects). Nevertheless, individuals keep appearing (11.3% of the total counted) with darker tones (*brown* and *red*), in which the insulator effect has not occurred. The protection against position effects is observed more clearly with the construct of the DNA binding sequence to the *SuHw* protein (BRwBR). In the 13 transgenic fly lines analysed most of the individuals maintained *orange, pale-orange* or *yellow* colours.

In the four HSwHS experimental constructs (10 transgenic fly lines analysed), XwX (8 transgenic flies analysed), LCRwLCR (21 transgenic fly lines analysed) and LCRmutwLCRmut (13 transgenic fly lines analysed) a clear displacement was observed towards lighter eye colours (*pale-orange* and *yellow*), especially in the HSwHS construct, the one with the greatest *insulator* capacity. From this experiment it can be concluded that the LCR sequence (SEC. ID. No. 1) off the mouse tyrosinase gene and its derivatives (HS, X and LCRmut) contain an insulator activity of the chromosomal position effects. This activity does not depend on the presence of the HS zone (boxes A and B) since in its absence (LCRmut) similar levels of insulation are obtained.

On the other hand, the results obtained in the comparative experiments carried out between the reference insulator sequence in vertebrates (5'HS4) and those of the invention reveal that the latter provide better protection against position effects in transgenic flies.

Table 1 summarises the results obtained with the gene constructs analysed in heterozygote transgenic flies, corresponding to the individuals shown in the bar diagram in Figure 6.

Table 2 summarises the results obtained with the gene constructs studied in homozygotic transgenic flies, corresponding to the individuals shown in the bar chart of Figure 7.

Evaluation of protection against position effects in homozygotic transgenic flies confirms the results obtained in heterozygotic transgenic individuals of the same lines, emphasizing the differences between sequences with insulator activity (BR, 5'HS4, LCR, LCRmut, HS and X) and the corresponding negative controls (w and TE3). Once again, the LCR DNA molecules and its derivates (LCRmut, HS and X, especially HS) present a better behaviour compared to DNA molecules with known insulator activity (BR and 5'HS4). In homozygosis, slight differences are observed between the LCR and LCRmut fragments (and between the HS and X fragments) that suggest that although the AB box is not essential to obtain insulator activity, its presence helps to organise the effective insulator element. The same transgenic fly lines have been analysed in heterozygosis (see Figure 6) and in homozygosis (see Figure 7) except for one line (of w), two (of TE3wTE3), two (of 5'HS4w5'HS4), one (of LCRwLCR), one (of LCRmutwLCRmut) and one (of BRwBR) for which it was not possible to obtain homozygote individuals.

**Table 1**

| **Summary of gene constructs analysed in heterozygotic transgenic mice** | | | | | |
|---|---|---|---|---|---|
| | *Red* | *brown* | *orange* | *pale orange* | *Yellow* |
| W | + | + | + | ++ | - |
| HSwHS | - | - | - | + | ++ |
| XwX | + | - | + | ++ | + |
| BRwBR | - | - | + | ++ | + |
| LCRwLCR | - | - | + | ++ | + |
| LCRmutwLCRmut | - | - | + | ++ | + |
| 5'HS4w5'HS4 | + | + | + | ++ | + |
| TE3wTE3 | + | + | + | ++ | + |

**Table 2**

| **Summary of gene constructs analysed in homozygotic transgenic mice** | | | | | |
|---|---|---|---|---|---|
| | *Red* | *brown* | *orange* | *pale orange* | *Yellow* |
| W | ++ | + | + | + | - |
| HSwHS | - | - | + | ++ | + |
| XwX | + | - | ++ | + | + |
| BRwBR | + | + | ++ | + | - |
| LCRwLCR | + | - | ++ | + | + |
| LCRmutwLCRmut | + | - | ++ | + | + |
| 5'HS4w5'HS4 | + | + | ++ | + | + |
| TE3wTE3 | + | + | ++ | + | + |

| | | | | | |
|---|---|---|---|---|---|
| Note: the category with the largest number of individuals has been identified in each case with two crosses (+). The presence of insulator activity is detected both by a reduced variability in eye pigmentation and also by increased presence of the lighter eye colour categories (*orange, pale-orange* and *yellow*). In homozygotic individuals eye pigmentation is proportionally intensified owing to the presence of two copies of the construct. [See figures 6 and 7 with the graphic representation of all the transgenic fly lines studied, both in heterozygosis (Figure 6) and in homozygosis (Figure 7)]. | | | | | |

### Example 3

### Generation of transgenic mice with the capacity to guarantee protection of an experimental gene construct against chromosomal position effects in the mouse genome

### Material and methods

### Constructs used in transgenic mice (see Figure 4).

pTLuc: The*SspI-BamHI* fragment of 1.8 kb obtained from the pD0432 plasmid (Rodriguez et al. 1988. Proc. Natl. Acad Sci. USA 85: 1667-1671) and that contains the luciferase gene, is cloned in the vector pW37SP1 (Montoliu et al., 1995. Proc. Natl. Acad. Sci. USA 92: 4244-4248) digested with *SmaI-Bg1II*, that bears the minimum promoter (*TATA* box, -35bp, of the thymidine kinase gene of HSV) together with two binding sites of the ubiquitous transcription factor Sp1 (Saffer et al., 1991. Mol. Cell. Biol. 11: 2189-2199; Marin et al., 1997. Cell 89: 619-628).
pHSTLuc: The 2.1 kb HS fragment was obtained from the plasmid pTyr14:E6 (Ganss et al., 1994. The EMBO Journal 13, 13: 3083-3093) digested with *XbaI-EcoRI* (see Figure 1) and is cloned in the *SaII* site of the pTLuc, via blunt ends in both DNA molecules.
pLCRTLuc: The *EagI-AflII* fragment of 3.0 kb obtained from the plasmid pTyr14: E6 (Ganss et al., 1994. The EMBO Journal 13,13:3083-3093), was cloned in the pHSTLuc fragment digested with identical restriction enzymes, in this way the HS fragment was completed to obtain the LCR (see Figure 1).

### The process to obtain transgenic mice

The transgenic mice were obtained by following the usual method (Hogan et al., eds. 1994. Manipulating the mouse embryo, CSHLP) by microinjection of fertilised oocytes of the albino strains NMRI or FVB/N (Harlan Iberica), with a DNA solution in microinjection buffer (10 mM Tris-HCl pH = 7.5, 0.1 mM EDTA pH = 8) at a concentration of 2 ng/µl. The oocytes were incubated for 16 hours, in a CO₂ incubator at 37ºC in M16 growth medium. Only embryos that passed to the following developmental stage (2 cells) were transferred to the oviducts of CD1 pseudopregnant females for gestation. The presence of DNA of the transgenic constructs in the genome of all the new born was studied by PCR, *slot-blot* and *Southern-blot* techniques. Estimation of copy number of the transgenic constructs integrated in the mouse genome was carried out in first generation individuals by (F₁) by *Slot-blot* using standards with known quantities (Ausubel et al., eds. 1990. Current Protocols in Molecular Biology, Green Publishing Associates and Wiley Interscience). The individuals identified as carriers of the transgene (founders) were crossed with NMRI albino mice to generate independent transgenic lines. A total of 5 independent lines were obtained and studied for the transgenes pTLuc and pHSTLuc and 8 for the pLCRTLuc transgene.

### Quantification of luciferase activity in transgenic mice (see Table 3 and Figures 5A, 5B and 5C).

To quantify luciferase activity in tissues, 6 adult mice were used (from 2 to 3 months old) of each line. Samples were extracted from the following tissues of each of these: skin from the dorsal or back region [BS], from both eyes [E], skeletal muscle [M], small intestine or thin gut [TG], kidney [K], spleen [S], liver [L], lung [N] and brain [B]. The abbreviations correspond to those used in Figures 5A, 5B and 5C. The tissues extracted were kept frozen at -80ºC until use.

To obtain protein extracts the tissues were homogenised in a homogeniser (Ultra-Turrax T8, IKA labortechnik) in 500 µl of lysis buffer (*Cell Culture Lysis Reagent,* Promega), the samples were centrifuged at Vₘₐₓ in 1.5 ml Eppendorf tubes for 5 minutes at 4ºC and the supernatant was transferred to a new tube, which was kept frozen at -80ºC until use.

Quantification of luciferase activity was done using protein extract (10µl) on white 96-well plates and by measuring the LCUs emitted with a luminometer (Microplate luminometer LB96V, EG&G Berthold) using as a reaction substrate 50µl of *Luciferase Assay Reagent* (Promega), following the manufacturer's instructions. To normalise the measurements, the protein concentration was assessed by mixing 2 µl of a 1/26 dilution of protein extract with 200 µl of the Bio-Rad protein assay (Bio-Rad) in 96-well plates and measuring absorbance at 620 nm in an Elisa reader (Titerket Multiskan MCC/340). The reference curve was drawn up with bovine y-globulin (Bio-Rad). The data are expressed as RLUs per microgram of protein, the value of each line in each tissue corresponds to the mean of each of the 6 individuals studied (+/- standard deviation).

In contrast to analogous experiments carried out with transgenic flies (see Example 2), in the case of transgenic mice, for obvious reasons, it was not possible to study so many constructs, independent lines and/or individuals. Nevertheless, as shown in the corresponding figure shown above, transgenic mice with three gene constructs were prepared: pTLuc, pHSTLuc and pLCRTLuc (Figure 4). The results obtained from analysis of transgene indicator activity (luciferase) in different tissues corresponding to 6 individuals per line are compiled in Table 3 and are shown in Figure 5, that represents luciferase activity measured in nine different organs of six individuals for each independent line of transgenic mice. The activities of the indicator transgene have been normalised relative to the total protein present in the protein extract in each case.

**Table 3**

| **Results of the analysis of luciferase activity** | |
|---|---|
| **pTLuc** | 5 independent lines, copy number: 2,3,4, (5) |
| | 4 express at low level in few tissues |
| **pHSTLuc** | 5 independent lines, copy number (1),(1), 1,2,3 |
| | 3 express at medium-high level in many tissues |
| **pLCRTLuc** | 8 independent lines, copy number: 1,5,5,8,7,13,21,187 |
| | 8 express at medium-high level in many tissues |

| | |
|---|---|
| NOTE: The lines for which expression was not detected are given in brackets. | |

The results obtained in transgenic mice show that the presence of the HS sequence and (better still) the LCR sequence can rescue multitissue expression of an experimental pTLuc transgene, comprised of a TATA box transcription start and two binding sites to a generalised expression transcription factor (Sp1)[Saffer et al., 1991, cited *supra;* Marin et al., 1997, cited *supra*],. The levels and presence of pTLuc transgene expression are very low and, therefore, sensitive to position effects. However, when HS and LCR sequences are added, transgenic mouse lines appear with expression of the transgene indicator in multiple tissues. The LCR sequences (SEC. ID. Nº: 1) merit a special mention since with these all the mouse lines obtained (8/8) are expressed to a high level in multiple organs.

From this experiment, it can be concluded that with the addition of HS sequences or, preferably, LCR sequences, expression of the experimental construct used can be guaranteed. The LCR sequence does not act as a mere specific transcriptional activator of tissue (in this case of the tyrosinase gene) since expression of the luciferase indicator gene is pronounced in tissues where endogenous expression of this gene does not occur (muscle, liver, brain etc. [see Figure 5]). For this reason, it can be concluded (analogously to the data obtained for transgenic flies [see Example 2]) that addition of the HS sequences or, preferably, LCR, makes the gene construct become independent from the site of integration in the genome, with expression of the indicator gene being detected in most (HS) or all (LCR) of the transgenic mouse lines studied. A clear relation is not observed between copy number and the expression level of the constructs (see Table 3 and Figures 5A, 5B and 5C), similar to when the 5'HS4 insulator sequence is used in transgenic mice (Potts et al., 2000, cited *supra*).

### EXAMPLE 4

### Comparative example

For comparative purposes, similar protection experiments against position effects are discussed in transgenic mice using an insulator sequence belonging to the state of the art (5'HS4) and the LCR insulator molecule of the invention (SEC. ID. No.:1). The results obtained show once again that the best relative results to guarantee expression of the transgenic mouse constructs are obtained with the LCR molecule.

The DNA molecule called LCR is used to improve the expression of tyrosinase minigenes in transgenic mice. Figure 3 of the article by Ganss et al., 1994 (cited *supra*) shows different transgenic mouse lines in which the LCR sequence has been added to a functional minigene of mouse tyrosinase (Ganss et al., 1994, cited *supra;* Monteliu L. 1994. Fade to grey. Current Biology 4: 1025). It was observed that the expression level achieved was higher, less variable (no observable variegation) and proportional to the copy number of the transgene inserted in the mouse genome (the pigmentation increased in intensity as more copies were integrated). It is worth noting that when these works were published the insulator activity from position effects of the LCR region was unknown.

When the transgenic mice mentioned in the previous paragraph were compared with the photographs used to illustrate the work by Potts et al. 2000 (cited *supra,* Figure 3 of this work) significant differences were observed between these. In this case too, tyrosinase transgenes are used to assess position effects, to which the known insulator sequence 5'HS4 is added. The presence of much weaker pigmentation intensities together with the greater variability and appearance of variegation in the second case suggest a better efficacy of the LCR sequence, an objective of this invention. The LCR sequence works better than the 5'HS4 sequence, although it must be taken into account that in this case LCR is the tyrosinase gene itself, that is used as a gene indicator. Similarly, it can be concluded that the LCR sequence is, at least, equally or more effective than the 5'HS4 sequence at protecting from position effects in transgenic mice. These data are corroborated by those obtained in transgenic mice with the pTLuc, pHSTLuc and PLCRTLuc constructs, which show that the presence of the HS or LCR sequence increases the guarantee of expression of the transgenes *in vivo.*

One advantage of the LCR sequence compared to the 5'HS4 sequence is that the latter (5'HS4) requires the presence of multiple elements (at least two units of the 1.2 kb element of the DNA sequence of the chicken β-globin gene) on each side of the experimental gene construct, a total of four), while the LCR sequence (and its smaller derivates) have manifest a protection activity against position effects in simple (transgenic mice) or double copy (5' and 3', in transgenic mice). The presence of one sequence repeated many times can cause problems of cloning and stability of the construct in bacteria and induce its inactivation in the host organism genome.

LIST OF SEQUENCES

<110> Consejo Superior de Investigaciones Científicas

<120> DNA MOLECULE WITH INSULATING ACTIVITY AGAINST
CHROMOSOMAL POSITION EFFECTS IN GENE TRANSFER PROCESSES IN
ANIMAL CELLS

<160> 1

<170> PatentIn version 2.0

<210> 1
<211> 3.711
<212> DNA
<213> MOUSE

<400> 1

gaattctgtc ctaaaacagt ctttgggctt tctttggggg gccggggggg atttggagtc 60
ttttaatgat tgcttttatt ttcttatata ttacaggtct atttaaattg ttaatatgat 120
tttcatttaa ctttggtaag tgatacctat caagaaaatt gtcatttcca attttatgaa 180
gtacaggttt ttgacatatg tgctagtaac tttctgtgtt tccttggtgt ctgctgttat 240
gttccctttt ggtttcaggt tttattgatt gggatagtct ctcagtgtct tttagttatt 300
tttgaataag agtttttcta ccttgttgat gttctcaaat aacctactct ttgtttcatt 360
gttaatgtgc attcttttgt ttctatttta ttggttccag gcatgagttt gattatttcc 420
tactttttac tcctcttggg tgtgtttgtt tctttttgtc ctagggattt tagctgtgct 480
attaagttgt tagtatgaga tgtctttaat ttctttacaa agacacttag tgctaagcac 540
ttttctcgta acaccacttt aactgtgttt gggtatatct tgcattcctt ttcattgaaa 600
tctggagagt cttttatttc tttctttatt tctgccttga cctcaaagtc atccagtagg 660
gagttgtgca gctttcatga gtttgtaggt tttctgttgt tgaatcccac ctttactcca 720
tgttgtctaa taagacacaa ggcattattt caactttctt atatctgttg aggcttattt 780
tgtgaacaac tatatgatca attttggaga aagtttaagg attgctgaga gaaaggtgcc 840
ttcttttttt gtttgggtga aatgttctaa aaaatatata ttaggtcttt tacctccatt 900
atttcttagt ttagtttttg tctagatgac atgtctattc atgagagtag gatattgaag 960
tctcccacta ttaatgtatt agattcaatt tgtgatttga gcattagtaa tgtttcactt 1020
ccaaatgtgt taccettgct ttggggacat agatgttaag aactgaagtg ttatcatggc 1080

taatttttcc tttgatgagt atgaaatgtc attttccatc tctattgact aattttcttg 1140
taaagtgtga tttgctagat attagaatag ctaaaacagc ttgcttcttg agtgcatctg 1200
cttggaatat ttttttcaa cccttaactg gaggta[?]tgt ctatcttgat g[?]tgaggtct 1260
attttttgta ctcagtgaaa tgattgattc tctttttgca tccactctgt ttgcctgtgt 1320
gttttattga gtaattgagt ccatttactt tgagaaatat taatgatcaa tgattcttca 1380
tttctgttat tttggtggtg gtggtgatag tagtgatagt tgtgtgcatg tgtatgtgtg 1440
tgtgtgtatg tgggtgtgtt tgatttttct ctgtatcatg attgcctata tgcaaaactg 1500
taaatatttt tcattaatac actagaattt gtgtttaaat gatatacaga ctaatctata 1560
ccaaatgtgt tattacagat tttctagact aaatgactca tcaatcctct gttagattta 1620
aattttgggt aactctttta tatatgtatc ctttataatg gatttagaag tttgaatccc 1680
atcaaaaaga cagtacatat atattgtatg gaagaatatc aaagttttgt agtgataaaa 1740
ctagccaatt ttggcctaga gctttgcagt gatgattagt agcaagagtt tggttgtagt 1800
aatatgttat cacactgtct ctctgaaa[?]g atatttctct aaatattcac ttctagcaat 1860
catgttcatt tg[?]ttacatg aaccaccaaa cctaaacatg aactacaaaa tgtatcaact 1920
ttcacccaca gtgttatttt ctcatggctc ttgtcctttg ttccaaattc aaccagttga 1980
aataatggca gtattttgta aattctgtac at[?]tttttc tctgttccct atacttgaca 2040
aaaatttccc tttttcaca ttcctctatt tacactgttc tttttca[?]g gttaagaaga 2100
gtcagtatga cccttcaagc agtgtttcct aacaatcttg tctcttaggc tcatttatag 2160
agggaatgag ctatgtctcc acaatacctc aatattactt gagaataaat tccaacatat 2220
agacctggta ataagccata agtaccttgt tccattgata agtgttcact tggacccatc 2280
agttactatt ccatacatct tcactttctg aacctcattt atgtttgaaa at[?]caagatt 2340
agaataacta tttaatggag gttatttaca aagatgcatt atgtggcatt tgtggtaaga 2400
atcatttcgt gggtccacaa atccattgca tcattccttg aagtggttta agtcttttga 2460
ttgtcattgt taataactgc attaaaagat tccttcattc cctgatttga gcttgacttt 2520
actcacagct agaaaattag aggcagaagg agagtgaagt caaaggtctg caacttcctc 2580
tttgccacat aacaagaaag ttgcttatat ccttctccaa ggtcatagtt cctgccagct 2640
gactttgtca agacagtgat gtctgtgttc cagcagttgt tctgagtatc cttttcatta 2700
tccactgtcc tttcttctta aattccaccc ccaacattgt aaatagcttc tttcttaaac 2760
tctg[?]tcaaa gaaccagctt gagtgtgtca gctgcttcct gctggggtcc tggcaaacca 2820
caatattatt aacaatatgt acccaataaa gagtcctgaa attgccacac catcaccttt 2880
taatattcct attcagattg tgaacttcta caaagcagga atgtggcttg tttattttcc 2940
tgttatcttg cactgtcaca cttaattctt gggaaactaa aaacacaaaa tatgcatttc 3000
tttggtctac tgaatgaaga tggaatgtg[?] attactctga ctctttaaat gtaaatggtg 3060
cttcatattc ctctaaagcc ttaagcacaa taacacaggg tgggtggtaa gaaaatatct 3120
gtagtatgga tacaaatttg ttccataaga atataactat aaaattatta aataaaatgt 3180
ttcaggaaaa tttaaactgg aacaaatcaa ggtaagtatc atctaaaaat gagcttaggt 3240
agacatgttg agttgagctt ctagggaagc tgttaagagt aattatttag aaaaaaagta 3300
ggtcaagaag aaaacactaa atagattatt cagataaaca ttttatttca aatagtattt 3360
tcatagaaca ttttgagggc atctgtttgg aaagaaattg attattgca[?] tagctctgag 3420
attcacatct ttcttgttta tataagagct cttttaaaat tgtgttttat gatactgtct 3480
cttgttgaat gttgcagtct ctcctccaat tcagacatct ccaagggtca ggaagcaaca 3540

tgagcaattc agtgaaagat agtatatttc caatgtctaa ccaaaaattg tagattctga 3600
tgaaagcctg gttgtgtgat gagacacaca gagagagaat cagacttgga aaatggctgc 3660
acagctacaa atacttcaaa tacttgctat caagcttaac ggcatgaatt c 3711

## Claims

1. A DNA molecule that consists of a nucleotide sequence selected from:
a) the nucleotide sequence identified as SEC. ID No. 1 or a fragment of this; and
b) a nucleotide sequence analogous to the sequence defined in a).

2. A DNA molecule according to claim 1, that presents insulator activity from the chromosomal position effects in gene transfer processes in animal cells.

3. A DNA molecule according to any of the previous claims that has the nucleotide sequence shown in SEC.ID. Nº1.

4. A DNA molecule according to either of claims 1 or 2, selected from the group comprised by the DNA molecules:
LCRmut: identical to SEC. ID. Nº1 except for a deletion of 56 bp between positions 2622 and 2683, inclusive, of SEC. ID. Nº1;
HS: comprised by the nucleotide sequence contained between nucleotide 1583 and 3711 of SEC.ID.Nº1; and
X: comprised by the nucleotide sequence contained between nucleotide 222 and 2617 of SEC.ID. Nº:1; and
A 1034 bp fragment that contains the nucleotide sequence contained between nucleotide 1583 and 2617 of SEC. ID. Nº1.

5. A DNA construct that contains (i) at least one DNA molecule according to any of claims 1 to 4 and, (ii) at least one transcription unit that contains one gene of interest, a promoter that directs transcription of this gene of interest and a transcription stop signal (polyadenylation).

6. A construct according to claim 6, that contains a first DNA molecule according to any of claims 1 to 4, in position 5' (*upstream*) of this transcription unit and a second DNA molecule according to any of claims 1 to 4, equal to or different from the first one, in tandem form with direct and/or inverted orientation.

7. A construct according to claim 6, that contains a first DNA molecule according to claims 1 to 4, in position 5' (*upstream*) of this transcription unit and a second DNA molecule according to any of claims 1 to 4, the same as or different to the former, with the same or the opposite orientation, in position 3' (*downstream*) of the gene of interest.

8. A vector comprised by a DNA construct according to any of claims 5 to 7.

9. A vector according to claim 8, selected from between a plasmid or a virus.

10. An animal cell that contains a DNA molecule according to any of claims 1 to 4, or a DNA construct according to any of claims 5 to 7, or a vector according to either of claims 8 or 9.

11. A transgenic non human animal comprised by, at least, one transgenic animal cell according to claim 10.

12. A kit comprised by, at least, one DNA molecule according to any of claims 1 to 4, or a DNA construct according to any of claims 5 to 7, or a vector according to either of claims 8 or 9.

13. A method to produce recombinant animal cells that consists in transforming animal cells with a DNA construct according to any of claims 5 to 7, or with a vector according to either of claims 8 or 9, under conditions in which these cells can be transformed.

14. A method to introduce transgenes in animal cells that consists in transforming animal cells with a DNA construct according to any of claims 5 to 7 or with a vector according to claims 8 or 9, under conditions in which these cells can be transformed.

15. A method to reduce the variability of transgene expression in animal cells that consists in transforming animal cells with a DNA construct according to any of claims 5 to 7, or a vector according to either claim 8 or 9, under conditions in which these cells can be transformed.
